# EUROPEAN PATENT APPLICATION

(11) **EP 1 916 006 A1**
(43) Date of publication of application: **30.04.2008**
(21) Application number: 06021957.3
(22) Date of filing: 19.10.2006
(51) Int. Cl.: A61L 31/08, A61L 31/14, A61L 27/14

(54) **Implant coated with a wax or a resin**

(71) Applicant: Schömig, Albert, 80638 München (DE); Wessely, Rainer, 85716 Unterschleissheim (DE); Kastrati, Adnan, 80638 München (DE); Wieczorek, Anna, 80638 München (DE)
(72) Inventor: Schömig, Albert, 80638 München (DE); Wessely, Rainer, 85716 Unterschleissheim (DE); Kastrati, Adnan, 80638 München (DE); Wieczorek, Anna, 80638 München (DE)
(74) Representative: Wichmann, Hendrik

(57) **Abstract**

The present invention relates to an implant or part thereof coated with a wax or a resin. Especially, the present invention relates to an implant or part thereof coated with shellac.

## Description

The present invention relates to a coated implant or part thereof, a method of preparing the same and a method of treating a subject, wherein the implant according to the invention is implanted into a subject.

Implants are artificial devices which made to replace or act as a missing biological structure. Additionally or alternatively, implants are used as a depot for substances such as drugs which are to be released to the surrounding tissue. This second type of implant may be used for e.g. brachytherapy, i.e. the placement of a substance such as a radioactive source in or near tissue to deliver e.g. radiation therapy.

Another particular type of implants are stents, which are used on diverse structures such as the oesophagus, trachea, or blood vessels. Prior to use, a stent is in general collapsed to a small diameter; when brought into place it is expanded using an inflatable balloon and is then held in place by its own tension. Stents are usually inserted by endoscopy or other procedures less invasive than a surgical operation, which makes them also suitable for patients with advanced disease for whom an operation might be too dangerous. Stents may consist of wire mesh alone, or may be coated by a suitable material.

Coronary stent implants are used for therapeutic cardiac procedures. Re-narrowing of a previously treated vascular lesion, termed restenosis, is considered the most important problem in interventional cardiovascular medicine. Stent placement has been shown to decrease the rate of restenosis. It involves placing a stent, which is a small mesh-like wire tube in a narrowed blood vessel. This procedure is similar to mere angioplasty in many ways. However, the stent is left permanently in place in the vessel to act as a scaffold to help keep the vessel open. Usually, an angioplasty procedure is performed first. The balloon inflations help open the vessel to allow the stent to be placed easily. The angioplasty catheter is removed. A different angioplasty catheter with a stent crimped on the balloon is advanced into the vessel and carefully positioned at the blockage. The angioplasty balloon is inflated which opens the stent and presses it into the vessel wall. The stent holds the vessel open and helps reduce the rate of restenosis (a recurrence of the narrowing within the vessel). In general the vessel is an artery.

Most of the implants (or at least a part of them) that are used today are made of bare-metal and/or polymeric material. Polymers are known to induce inflammatory responses which can translate into delayed healing and thus increased risk for an adverse outcome.

Furthermore, also bare metal implants are associated with side-effects. It has been shown that up to 40 % of patients who received a bare-metal stent develop in-stent restenosis.

The risk of adverse reactions or side effects can be reduced by additional administration of suitable drugs preventing the same. For this, implants releasing drugs which reduce adverse reactions or side effects have been developed.

For example, in-stent restenosis can be substantially reduced by the implantation of a drug-eluting stent (DES) (Babapulle et al., 2004, The Lancet 364: 583 - 591). Currently, two devices are approved by the FDA, both of which have shown efficacy towards the prevention of restenosis, the Cypher^{®} stent (Cordis, Johnson&Johnson), which is coated with rapamycin (Moses et al., 2003, N Eng J Med 349: 1315 - 1323) and the Taxus^{®} stent (Boston Scientific) (Stone et al., 2004, N Engl J Med 350: 221 - 231) that releases paclitaxel.

To protract the release of the compound, both devices make use of a polymeric coating. While this choice of coating is effective, it imposes several risks which may limit the clinical outcome, since the used polymers are known to induce inflammatory responses as detailed above. In deceased patients who previously received a polymer coated drug-eluting stent, ongoing inflammation and impeded endothelialization could be observed consistently. Additionally, there are concerns that polymer-coated DES implanted in patients may increase the chance for late stent thrombosis as well as late in-stent restenosis by means of chronic adverse pathomechanisms, mainly induced by the polymer.

To circumvent the need for polymeric coating, a drug-eluting stent platform that uses a microporous stent surface that serves as "drug pockets" to delay the release of a given drug has been recently introduced (Wessely et al., 2005, Arterioscler Thromb Vasc Biol 25: 748 - 753). This DES system liberates two thirds of the drug within the first week. The system proved safe and effective both in a standard pre-clinical model as well as in humans.

However, it is believed that the protracted drug release of the polymer-coated stents described above, which liberate the drug within 60 days, is responsible for the clinical results concerning in-stent restenosis at 6-9 months follow up. Therefore, in certain clinical situations a slower release kinetic is required. Modulation of release kinetics of an implant-based, e.g. stent-based, compound to prevent e.g. in-stent restenosis is crucial to improve clinical efficacy. Most current drug-eluting stent platforms use polymeric coating to achieve protracted drug liberation; however, potential life-threatening risks have been identified for polymers, such as late stent thrombosis. Therefore, retardation of drug release from the stent platform has to be achieved by an alternative mechanism.

Therefore, there is a need for further implants for use e.g. in cardiac diseases.

The present invention relates to an implant or a part thereof coated with a wax or a resin.

An implant according to the invention may be any implant made by man and known in the art. Therefore, preferably said implant of the invention is designated as "artificial". An implant is therefore an artificial device which is made e.g. to replace and/or act as a missing biological structure, to support a deleftive structure or as a depot for a substance. There is a multitude of different implants which replace different functions in humans. Modem medical implants are generally high-end devices. The complete implant or its outer shell is in general made of a bioinert material, in most cases from metal such as titanium. In some cases implants contain electronics e.g. artificial pacemakers and cochlear implant. In other cases the medical implant has compound structure and/or acts as reinforcement e.g. dental implant, knee joint replacement implant.

According the invention, a part of an implant means any part which can be separated from the implant. Especially, if the implant is comprised of several individual part, the term "part" refers to one of these parts.

In a preferred embodiment of the invention, the implant comprises metal. In a preferred embodiment, the implant apart from the coating is entirely made of metal.

It is also envisaged within the present invention that the implant comprises metal and one or more other compounds (e.g. as described below) or is made entirely of one or more of these compounds.

Examples of metals suitable for implants include stainless steel, cobalt, chromium, Nickel-titanium shape memory metal Nitinol, and titanium or alloys comprising or consisting of these. Examples of such alloys are titanium- or cobalt/chromium-based alloys.

Additionally, as discussed above, the implant may comprise or may be made of other materials such as ceramic materials (aluminium oxide or zirconium oxide) or polymeric materials such as polyethylene, polyglycolic-polylactic acid, polyethyleneoxidepolybutylene, terephthalate, polyorthoester, Biogold polyamine-heparin, methacryloylphosphorylcholine-laurylmethacrylate polycaprolactone, polyethylene terephthalate, silicone and polyurethane.

The implant may be composed of different layers, such as a core, e.g. a ceramic core, and a can, such as titanium can. Additionally, implants may be coated with biological materials such as fibrin, cellulose or albumin. However, implants without non-naturally occurring polymeric surface materials are preferred.

Examples of implants include, without limitation, pacemakers or heart valves, metal implants in a human brain, eye or ears, dental implants, orthopaedic implants such as hip and knee replacements; plates, screws, and rods used to treat fractures, drug release systems, cavity fillings, orthopaedic devices, stents, self-expanding endoluminal prosthesis sleeves for stent delivery, finger joints, and coronary artery bypass grafts etc.

In one preferred embodiment of the invention the implant is a valve, a dental implant or an orthopaedic implant system.

In another preferred embodiment of the invention the implant is a stent. According to the invention, a stent is a tube made of e.g. metal or other compounds as described above that may be inserted into a vessel or passage to keep it open and prevent closure due to a stricture or external compression, e.g., to keep an artery open after a heart attack.

Any stent known to the skilled practitioner may be used. Preferably, the stent is a bare metal stent with or without surface modification. Examples of stents, which are commercially available from e.g. Boston Scientific (Natick, MA, USA), Medtronic Inc. (Minneapolis, MN, USA), Abbott Laboratories (Abbott Park, IL, USA), Guidant Corporation (Indianapolis, IN, USA), Johnson & Johnson (New Brunswick, NJ, USA), Biometric GmbH & Co. KG (Berlin, Germany) or OptiMed Technologies, Inc. (Fairfield, NJ, USA), include - without limitation:
- Bx VELOCITY® Coronary Stent (Johnson & Johnson)
- S.M.A.R.T.® CONTROL™ Stent Delivery System (Johnson & Johnson)
- PRECISE® Nitinol Self-expanding Stent (Johnson & Johnson)
- PALMAZ® CORINTHIAN™ Transhepatic Biliary Stent and Delivery System (Johnson & Johnson)
- Driver™ Coronary Stent (Medtronic)
- Racer Stent (Medtronic)
- Liberté Coronary Stent System (Boston Scientific)
- Magic WALLSTENT® stent (Boston Scientific)

According to the invention, the implant or part thereof is coated with a wax or a resin.

Preferably, the wax or resin is biocompatible (i.e. acceptable for application to human beings) and /or erodable, i.e. slowly degraded in the human body over several weeks, months or years.

The invention includes that the stent is either coated with a wax or resin or coated with both.

According to the invention, the term "coated" or "coating" means that the implant or part thereof has a layer (either at its surface or below another coating) comprising the given material or the given materials. In general, the thickness of the coating will be in the range of 5 to 15 µm, although the skilled person will appreciate that also other thicknesses could be used.

Methods for coating of artificial implants are known in the art (Wessely et al., 2005, Arterioscler Thromb Vasc Biol 25: 748 - 753) and are discussed below.

According to one aspect of the invention, the implant or part thereof is coated with a wax.

A wax (see also Römpp Lexikon Chemie, Georg Thieme Verlag Stuttgart, 10th edition 1996, p. 4906, "Wachse", incorporated herein by reference) may be a substance which is plastic (malleable) at normal ambient temperatures, has a melting point above approximately 45 °C (113 °F), a relatively low viscosity when melted (unlike many plastics), is insoluble in water and hydrophobic.

Preferably, a wax is an ester of a mono- or diol with fatty acids, preferably long-chain fatty acids.

Preferably, the wax is selected from the group of plant derived, animal derived or geologically derived waxes, respectively. The wax may further be chemically modified or synthetic.

According to a further aspect of the invention, the natural product is a resin.

A definition for the term "resin" may also be retrieved from Römpp Lexikon Chemie (see above, p. 1692, "Harze", incorporated herein by reference).

In general, a resin may be a hydrocarbon secretion of many plants, particularly coniferous trees, valued for its chemical constituents and uses such as varnishes and adhesives. However, this term is also used for synthetic substances of similar properties.

Resin as produced by most plants may be a viscous liquid, typically composed mainly of volatile fluid terpenes, with lesser components of dissolved non-volatile solids which make resin viscous and sticky. The most common terpenes in resin are the bicyclic terpenes alpha-pinene, beta-pinene, delta-3 carene and sabinene, the monocyclic terpenes limonene and terpinolene, and smaller amounts of the tricyclic sesquiterpenes longifolene, caryophyllene and delta-cadinene. The individual components of resin can be separated by fractional distillation. Some resins contain high proportion of resin acids.

A few plants produce resins with different compositions, most notably Jeffrey Pine and Gray Pine, the volatile components of which are largely pure n-heptane with little or no terpenes. These resins may also be used in the contextof the present invention.

Some resins when soft are known as oleo-resins, and when containing benzoic acid or cinnamic acid they are called balsams. Other resinous products are in their natural condition mixed with gum or mucilaginous substances and known as gum resins. Many compound resins have distinct and characteristic odours, from their admixture with essential oils.

The hard transparent resins, such as the copals, dammars, mastic and sandarac, are principally used for varnishes and cement, while the softer odoriferous oleo-resins (frankincense, elemi, turpentine, copaiba) and gum resins containing essential oils (ammoniacum, asafoetida, gamboge, myrrh, and scammony) are more largely used for therapeutic purposes and incense.

According to the invention, the term resin may also include natural or synthetic derivatives of the resins mentioned above.

This may include a solidified resin from which the volatile terpene components have been removed by distillation is known as rosin. Typical rosin is a transparent or translucent mass, with a vitreous fracture and a faintly yellow or brown colour, non-odorous or having only a slight turpentine odour and taste. It is insoluble in water, mostly soluble in alcohol, essential oils, ether and hot fatty oils, softens and melts under the influence of heat, is not capable of sublimation, and bums with a bright but smoky flame. This comprises a complex mixture of different substances including organic acids named the resin acids. These are closely related to the terpenes, and derive from them through partial oxidation. Resin acids can be dissolved in alkalis to form resin soaps, from which the purified resin acids are regenerated by treatment with acids. Examples of resin acids are abietic acid (sylvic acid), C₂₀H₃₀O₂, plicatic acid contained in cedar, and pimaric acid, C₂₀H₃₅O₂, a constituent of gallipot resin. Abietic acid can also be extracted from rosin by means of hot alcohol; it crystallizes in leaflets, and on oxidation yields trimellitic acid, isophthalic acid and terebic acid. Pimaric acid closely resembles abietic acid into which it passes when distilled in a vacuum; it has been supposed to consist of three isomers.

Synthetic resins are also included in the context of the present invention and are materials with similar properties to natural resins-viscous liquids capable of hardening. They are typically manufactured by esterification or soaping of organic compounds. The classic variety is epoxy resin, manufactured through polymerization-polyaddition or polycondensation reactions, used as a thermoset polymer for adhesives and composites. One more category, which constitutes the 75% of resins used, is Unsaturated Polyester Resin. Ion exchange resin is another important class with application in water purification and catalysis of organic reactions.

Preferably, the resin may be selected from the group consisting of recent resins, recent fossile resins, semi-fossile resins and fossile resins.

Most preferably, the resin is shellac (for definition see also Römpp Lexikon Chemie (see above), p. 3955 to 3956, "Schellack", incorporated herein by reference). As demonstrated in the attached examples, drug release is significantly retarded in stents coated with shellac and rapamycin.

Shellac is a brittle or flaky secretion of the lac insect *Coccus lacca,* found in the forests of Assam and Thailand. When purified, the chemical takes the form of yellow/ brown pellets, this possibly providing the basis for the "Wing Source Story." Shellac is a natural polymer and is chemically similar to synthetic polymers, thus it is considered a natural plastic.

It can be molded by heat and pressure methods. It is soluble in alkaline solutions such as ammonia, sodium borate, sodium carbonate, and sodium hydroxide, and also in various organic solvents. When dissolved in alcohol, typically blends containing ethanol and methanol, shellac yields a coating of superior durability and hardness and is available in numerous grades. Shellac refined for industrial purposes may either retains its natural wax content or is refined wax-free by filteration. Orange shellac is bleached with sodium hypochlorite solution to form white shellac and also is produced in wax-containing and wax-free form.

Shellac comprises, as main constituents, aleuritic acid and shellolic acid. However, the term "shellac" also comprises analogues and derivatives of shellac as discussed above which may comprise analogues and derivatives of these acids.

Therefore, in a preferred embodiment, the present invention relates to a stent coated with shellac.

According to a further preferred embodiment of the invention, the implant or part thereof is further coated polylactide, preferably with poly-DL-lactide. However, it is also included within the present invention that the implant or part thereof is further coated with a poly-D-lactide or a poly-L-lactide. Polylactides are commercially available, e.g. from Boehringer Ingelheim.

According to the invention, polylactide or poly lactic acid is a polyester on basis of lactic acid, which can be produced starting from its lactide by ring opening polymerization. Polylactide or poly lactid acid, lactide and lactic acid are terms known to the person skilled in the art.

As demonstrated in example 2, the combined coating of shellac and poly-DL-lactide results in a significant retardation of drug release in comparison to a coating with poly-DL-lactide alone.

Consequently, in a preferred embodiment, the invention relates to a stent coated with shellac and polylactide, preferably poly-DL-lactide.
According to a further embodiment, the implant is further coated with a compound. In general, a preferred compound is a drug, i.e. a substance or a mixture of substances intended and/or suitable for use in the diagnosis, cure, mitigation, treatment or prevention of disease, disorder, abnormal physical state, or the physical symptoms thereof, in a human or animal receiving the implant. This includes e.g. a drug which is protein on nucleic acid based, e.g. an antibody..

Examples of drugs include, but are not limited to, anti-infectiva such as antibiotics and antiviral agents, analgesics and analgesic combinations, anorexics and appetite suppressants, anthelmintics, anesthetics, antiarthritics, antiasthma agents, anticonvulsants, antidepressants, antidiabetic agents, antidiarrheals, antihistamines, anti-inflammatory agents, antimigraine preparations, antimotion sickness agents, antinauseants, antineoplastics, antiparkinsonism agents, antipruritics, antipsychotics, antipyretics, antispasmodics, anticholinergics, sympathomimetics, xanthine derivatives, cardiovascular preparations including calcium channel blockers, beta blockers, antiarrhythmics, antihypertensives, diuretics, vasodilators (general, coronary, peripheral and cerebral), central nervous system stimulants, cough and cold preparations, decongestants, diagnostics, hormones, hypnotics, immunosuppressives, muscle relaxants, parasympatholytics, parasympathomimetics, psychostimulants, sedatives, tranquilizers, antioxidants, vitamins, minerals, and herbal extracts or preparations.

Preferred examples of drugs, particularly for implants into the cardiovascular system, include but are not limited to: anti-inflammatory drugs, antiproliferative drugs, antibiotics, antithrombotics, ACE inhibitors, β blockers, diuretics, cardiac glycosides, angiotensin receptor blockers, PDE blockers, vasodilators, antiarrhythmics, anticholinergics, benzodiazepines, calcium channel blockers, anticoagulants, salicylates or antihistamines.

Most preferably, the stent is coated with rapamycin or an analogue thereof as defined below. As detailed above, the coronary artery response to stent implantation leads to a complex and largely predictable sequence of events related to inflammation and repair processes including thrombosis disturbances (acute phase) and tissue remodelling (chronic phase). Interestingly, neither systemic application of anticoagulants, anti-inflammatories, nor anti-proliferative drugs have consistently prevented restenosis. Therefore, it is desirable to coat the stent with a drug having at least one of the following properties:
- Antithrombotic
- Anti-inflammatory
- Anti-proliferative
- Non toxic
- Effect only on multiplying cells
- Promotes or at least does not interfere with re-endothelialization
- No late thrombosis

In another preferred embodiment of the invention the drug is an inhibitor of mTOR (mammalian target of rapamycin). Examples of such inhibitors are listed and described below.

In still another preferred embodiment the drug is a macrolide. Macrolides are a group of drugs (typically antibotics) whose activity stems from the presence of a macrolide ring, a large lactone ring to which one or more deoxy sugars, usually cladinose and/or desosamine, are attached. The lactone ring can e.g. be either 14, 15 or 16-membered. The macrolide may be any natural occuring macrolide, e.g. those produced by various strains of Streptomyces, or a derivative thereof.

More preferably, the macrolide is rapamycin or an analogue thereof.

In the context of the present invention the terms "derivative" and "analogue" are used interchangeably. These terms refer to chemical modifications of the respective basic molecule. Chemical modifications are known to the skilled person and include e.g. hydroxylation, alkylation, esterification, oxidation, hydration, addition or removal of groups such as alkyl, alkenyl, alkinyl, aryl, aralkyl amino, sulfhydryl, hydoxy, fluoro, bromo, carboxy and/or amino groups etc. The basic molecule may be modified in order to maintain or improve the controlling, e.g. protracting, properties of the resulting molecule. The properties can be tested by the test detailed in the examples, in order to determine the resulting kinetics of drug release.

Rapamycin is a naturally-occurring macrolide antibiotic produced by the fungus *Streptomyces hygroscopicus,* found on Easter Island. The name is derived from Rapa Nui, the native name for Easter Island. Rapamycin is a hydrophobic synthetic drug, FDA-approved as an oral immunosuppressive agent used to prevent organ transplant rejection. Rapamycin (also known as Sirolimus) is an excellent candidate for the treatment of AV graft stenosis, since it significantly reduces in-stent restenosis and prevents chronic organ rejection. It blocks T-cell activation and cytokine and growth factor-mediated smooth muscle cell proliferation.

Being a hydrophobic drug, it dramatically increases local (vessel wall) concentrations, while allowing exquisite control over release kinetics. Because local drug concentrations are inextricably linked to biological effects, and not mere proximity, rapamycin appears as an ideal anti-restenotic drug. Rapamycin exhibits the following pharmacochemical advantages:
- Cytostatic, not cytotoxic
- Readily diffuses across vascular tissue
- It achieves high local tissue concentration
- Inhibits the cell cycle, which constitutes a key target for antirpoliferative therapies
- Long half-life in tissues
- Inhibits inflammation by blocking local cytokines
- Potent (only microgram doses required)
- Safe in humans at blood concentrations far exceeding dose delivered from stents

However, analogues of rapamycin are also useable in the context of the present invention. Examples include (without limitation) those disclosed in EP1413581 such as: 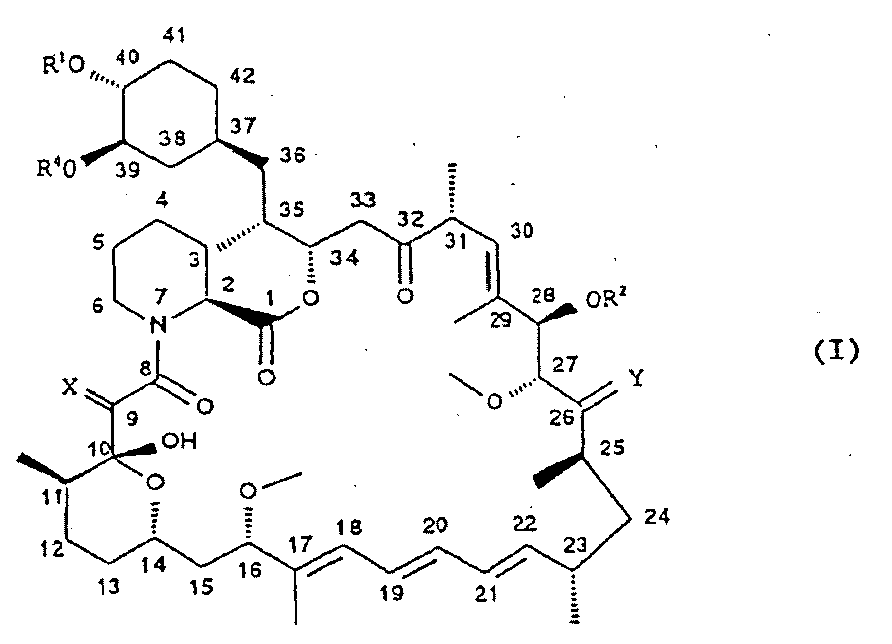 wherein
X is (H,H) or O;
Y is (H,OH) or O;

R¹ and R² are independently selected from H, alkyl, thioalkyl, arylalkyl, hydroxyalkyl, dihydroxyalkyl, hydroxyalkylarylalkyl, dihydroxyalkylarylalkyl, alkoxyalkyl, acyloxyalkyl, aminoalkyl, alkylaminoalkyl, alkoxycarbonylaminoalkyl, acylaminoalkyl, arylsulfonamidoalkyl, allyl, dihydroxyalkylallyl, dioxolanylallyl, carbalkoxyalkyl, and (R³)₃Si where each R³ is independently selected from H, methyl, ethyl, isopropyl, t-butyl, and phenyl; wherein "alk-" or "alkyl" refers to C1-6 alkyl, branched or linear, preferably C1-3 alkyl, in which the carbon chain may be optionally interrupted by an ether (-O-) linkage; and
R⁴ is methyl or R⁴ and R¹ together form C2-6 alkylene;
provided that R¹ and R² are not both H; and provided that where R¹ is carbalkoxyalkyl or (R³)₃Si, X and Y are not both O.

Preferred examples of these analogues are:
40-O-Benzyl-rapamycin
40-O-(4'-Hydroxymethyl)benzyl-rapamycin
40-O-[4'-(1,2-Dihydroxyethyl)]benzyl-rapamycin
40-O-Allyl-rapamycin
40-O-[3'-(2,2-Dimethyl-1,3-dioxolan-4(S)-yl)-prop-2'-en-1'-yl]-rapamycin
(2'E, 4'S)-40-O-(4',5'-Dihydroxypent-2'-en-1'-yl)-rapamycin
40-O-(2-Hydroxy)ethoxycarbonylmethyl-rapamycin
40-O-(2-Hydroxy)ethyl-rapamycin
40-O-(3-Hydroxy)propyl-rapamycin
40-O-(6-Hydroxy)hexyl-rapamycin
40-O-[2-(2-Hydroxy)ethoxy]ethyl-rapamycin
40-O-[(3S)-2,2-Dimethyldioxolan-3-yl]methyl-rapamycin
40-O-[(2S)-2,3-Dihydroxyprop-1-yl]-rapamycin
40-O-(2-Acetoxy)ethyl-rapamycin
40-O-(2-Nicotinoyloxy)ethyl-rapamycin
40-O-[2-(N-Morpholino)acetoxy]ethyl-rapamycin
40-O-(2-N-Imidazolylacetoxy)ethyl-rapamycin
40-O-[2-(N-Methyl-N'-piperazinyl)acetoxy]ethyl-rapamycin
39-O-Desmethyl-39,40-O,O-ethylene-rapamycin
(26R)-26-Dihydro-40-O-(2-hydroxy)ethyl-rapamycin
28-O-Methyl-rapamycin
40-O-(2-Aminoethyl)-rapamycin
40-O-(2-Acetaminoethyl)-rapamycin
40-O-(2-Nicotinamidoethyl)-rapamycin
40-O-(2-(N-Methyl-imidazo-2'-ylcarbethoxamido)ethyl)-rapamycin
40-O-(2-Ethoxycarbonylaminoethyl)-rapamycin
40-O-(2-Tolylsulfonamidoethyl)-rapamycin
40-O-[2-(4',5'-Dicarboethoxy-1',2',3'-triazol-1'-yl)-ethyl]-rapamycin

Further examples of rapamycin analogues included:
- SDZ RAD (RAD001; 40-O-(2-hydroxyethyl)-rapamycin; everolimus; Novartis International AG, Basel, CH),
- SAR 943 (32-Deoxorapamycin; Sedrani et al., 1998, Transplant Proc 30:2192-2194)
- ABT 578 (zotarolimus; Abbott Laboratories, Abbott Park, IL, USA),
- CCI-779 (temsirolimus; Wyeth-Ayerst Research Laboratories, Princeton, NJ, USA),
- AP20840 (ARIAD Pharmaceuticals, Cambridge, MA, USA)
- AP23573 (ARIAD Pharmaceuticals, Cambridge, MA, USA)
- AP21967 (Pollock et al., 2002, Nature Biotechnology 20, 729 - 733),
- AP22565 (Pollock, R. et al., 2000, Proc. Natl. Acad. Sci. USA 97, 13221-13226),

However, most preferably, rapamycin is used as drug, particularly for cardiovascular implants such as stents.

In another preferred embodiment of the invention, the drug is an adrenoceptor antagonist, particularly a β blocker, or a steroid.

The feature "adrenoreceptor antagonists" relates to a group of substances capable of partially or fully inhibiting adrenergic receptors. Adrenergic receptors (or adrenoceptors) are a class of G protein-coupled receptors that is the target of carecholamines. Adrenergic receptors specifically bind their endogenous ligands, the catecholamines adrenaline and noradrenaline (also called epinephrine and norepinephrine) and are activated by these. The class of adrenergic receptors is subdivided into two subclasses, α- and β-receptors, wherein each subclass comprises several subtypes (α₁-, α₂-, β₁-, β₂- and β₃-adrenocepor). In general adrenergic blockers mimic the structure of a catecholamines, however, replacing the catechol ring with a bulky substituent (e.g. propranolol) makes it likely that the resulting compound will block adrenoceptors, as the interactions on loop V of the receptor are important to agonist activity.

α-Blockers, also known as α-adrenergic blockers, are used to treat high blood pressure and other conditions like an enlarged prostate. Examples of α blockers are phenoxybenzamine, phentolamine, phenoxybenzamine, prazosin, doxazosin, terazosin, tamsulosin, yohimbine, idazoxan,

β-Blockers, also known as β-adrenergic blockers, are used to treat e.g. high blood pressure (hypertension), congestive heart failure (CHF), abnormal heart rhythms (arrhythmias), and chest pain (angina). β-Blockers are sometimes used in heart attack patients to prevent future heart attacks. They constitute a heterogeneous class of agents having a different degree of selectivity of β₁-adrenergic receptors and the presence of additional properties.

Based on these characteristics, β-blocking agents have been categorized into three classes. First generation agents, such as propranolol and timolol, block both β₁- and β₂-receptors and do not exhibit any particular property. These agents are not well tolerated in heart failure because they exhibit negative inotropic effects associated with an increase in peripheral vascular resistance, secondary to (β₂-blockade. Second generation agents, such as metoprolol, atenolol, and bisoprolol produce a selective β₁-blockade and do not have additional properties. They do not increase peripheral vascular resistance and are better tolerated by heart failure patients. However, acute administration may produce a decline in cardiac output and an increase in ventricular filling pressure due to negative inotropic effects. Third generation agents, such as bucindolol and carvedilol, are not selective but exhibit ancillary properties, which may be important to their tolerability and efficacy in heart failure patients

Examples of β-blockers are propranolol, alprenolol, oxprenolol, metoprolol, acebutolol, atenolol, betaxolol, bisoprolol, carteolol, carvedilol, labetalol, nadolol, penbutolol, pindolol, sotalol and timolol.

The most preferred β-blocker is carvediolol or an analogue thereof, particularly if rapamycin is the first drug. Carvedilol is a vasodilating β-blocker and antioxidant approved for treatment of mild to moderate hypertension, angina, and congestive heart failure. SB 211475, a hydroxylated carvedilol analogue, is an even more potent antioxidant in several assay systems.

In a further preferred embodiment of the invention the drug is a steroid. Steroids are cholesterol derivatives and have tetracyclic basic structure consisting of four fused rings: three cyclohexane rings and one cyclopentane. Rings A and D of steroids are the most commonly modified rings.

Steroids have a variety of uses in the human body, including, but not limited to: controlling meiosis, carbohydrate metabolism, fat storage, muscle growth, immune function and nerve cell membrane chemistry. Steroids can also be divided into groups by function: androgens, estrogens, estradiol, progestogens, anabolics, and catabolics. The gonadal variety is mainly synthesized in the gonads, as is suggested by the name, while the glucocorticoids (e.g. cortisol, cortisone) and mineral corticoids (eg aldosterone) are synthesized in the adrenal cortex.

Examples of steroids are: cholesterol, estradiol, testosterone, cortisone, cortisol, corticostreone, aldosterone, progesterone, 7-dehydocholesterol, cholic acid, pregnenolone, dihydrotestosterone, nandolone, dihydoandrolone, androdiol, oxandolone and anadrol.

More preferably, the second drug is estradiol or an analogue thereof. The analogue is as defined above. Examples of estradiol analogues are: 3-Methoxy-17b-hydroxyestra-1,3,5(10)-triene, 3-Acetoxy-17b-hydroxyestra-1,3,5(10)-triene, 3,17b-Diacetoxyestra-1,3,5(10)-triene, 3-Propionoxy-17b-hydroxyestra-1,3,5(10)-triene, 3,17b-Dipropionoxyestra-1,3,5(10)-triene, 3-Benzoyloxy-17b-hydroxyestra-1,3,5(10)-triene, 3,17b-Dibenzoyloxyestra-1,3,5(10)-triene, 3-Acetoxy-17b-benzoyloxyestra-1,3,5(10)-triene, 3,17b-Dihydroxyestra-1,3,5(10)-triene-6-one (6-oxoestradiol), 3-Methoxy-17b-hydroxyestra-1,3,5(10)-triene-6-one, 3-Hydroxy-17b-propionoxyestra-1,3,5(10)-triene-6-one, 3,17b-Dipropionoxyestra-1,3,5(10)-triene-6-one, 3,9a-Dihydroxy-17b-propionoxyestra-1,3,5(10)-triene-6-one, 3,17b-Dipropionoxy-9a-hydroxyestra-1,3,5(10)-triene-6-one. An even more preferred derivative of estradiol is 17β estradiol valerate.

The invention further relates to a method of preparing the implant according to the present invention defined and described above, wherein an implant, preferably an uncoated implant is coated with the natural product.

The implant may be coated by any procedure suitable for coating a solid material with a solution. Scu methods are known in the art and are e.g. described in Wessely R. et al., 2005, Arterioscler Thromb Vasc Biol 25: 748 - 753.

In general, drug or resin and / or wax or both combined are dissolved in an appropriate solvent which is subsequently transferred to a drug reservoir. The latter is attached to the coating machine. Thereafter, the coating process is initialized by spray coating. Coating patterns can be optimized by appropriate adjustments of coating velocity and nozzle spray pressure. Importantly, the coating process can also encompass two or more subsequent coatings, e.g. a base and a top coating process.

Thereafter, the implant is dried e.g. by mild heating, air-drying or any other suitable method known to the person skilled in the art.

In a preferred embodiment of the invention the uncoated implant is spray-coated. Even more preferably, the coating machine described by Wessely and coworkers is used, which permits individual, on-site coating of implants such as stents e.g. with a unique microporous surface (Wessely R. et al., 2005, Arterioscler Thromb Vasc Biol 25: 748 - 753) allowing for individualizable, dose-adjustable, and multiple coatings with identical or various compounds, designated ISAR (individualizable drug-eluting stent system to abrogate restenosis).

In a preferred embodiment of the method of the invention, the implant or part thereof is further coated with a drug as defined above. Preferably, this is performed by spray coating.

In another aspect, the present invention provides the implant or part thereof of the invention for use in prophylaxis or therapy. In a preferred embodiment, the implant or part thereof, preferably a stent, is for use in the prophylaxis or therapy of a cardiac disease or vascular disease, preferably a stenosis or restenosis. However, other diseases where local drug delivery might be important could also be treated.

Still another aspect of the invention relates to a method of treating a subject, wherein an implant, preferably a stent, according the present invention and as defined and described above is implanted into a subject.

Methods of implanting an implant into a subject such a human or an animal are known to the skilled practitioner.

If a stent is to be implanted in the context of cardiac diseases, the following procedure may be carried out: the stent is collapsed to a small diameter and put over a balloon catheter. It is then moved into the area of the blockage. When the balloon is inflated, the stent expands, locks in place and forms a scaffold. This holds the vessel open. The stent stays in the vessel permanently, holds it open, improves blood flow to the heart muscle and relieves symptoms (usually chest pain).

The invention is further characterized by the following figures and examples which are included for informational purposes and which are not intended to limit the scope of the claims.

### Short description of the Figures

**Fig.1**
   Microscopic appearance of a stent coated with a mixture of 0.5% shellac and 1.0% rapamycin dissolved in ethanol p.a. A smooth layer is visible without apparent clumping or webbing of the shellac/rapamycin blend.
**Fig.2.**
   In vitro release kinetics of stents coated with 1.0% rapamycin without shellac blend (X) and 1.0% rapamycin/0.5% shellac composite. Shellac has a profound impact on stent-based rapamycin by protracting drug release long-term.
**Fig. 3.**
   Drug concentration of rapamycin *in vivo* three weeks after stent placement in porcine coronary arteries. Whereas the concentration of rapamycin in the vascular wall is marginal for stents solely coated with rapamycin (left), it is appreciably higher after placement of stents coated with shellac (middle). Stents coated with rapamycin and shellac show intravascular rapamycin concentrations similar to what can be measured after placement of the polymer coated, rapamycin-eluting Cypher™ stent (right).
**Fig. 4.**
   In vitro release kinetics of stents coated with a solution containing 0.4 % rapamycin (4 mg/ml) and 0.7 % poly-DL-lactide (Resomer R 202 S, Boehringer Ingelheim) (7 mg/ml) in ethyl acetate ("polymer coating"), a solution containing rapamycin alone in ethyl acetate ("rapamycin only, no further coating") or with a solution containing 0.4 % rapamycin (4 mg/ml) and 0.7 % poly-DL-lactide and a solution of 0.1 % rapamycin (1 mg/ml) and 0.5 % shellac (5 mg/ml) in ethanol ("shellac + polymer coating"). Both poly-DL-alone as well as a combination of poly-DL-lactide and shellac have a profound impact on stent-based rapamycin by protracting drug release long-term.

### Example 1

### 1. Introduction

Re-narrowing of a previously treated vascular lesion, termed restenosis, is still considered the most important problem in interventional cardiology (1). Stent placement has been shown to decrease the rate of restenosis (2), however, the problem remains substantial with in-stent restenosis developing in up to 40% of patients who received a bare-metal stent (3). The risk of in-stent restenosis can be substantially reduced by the implantation of a stent that elutes compounds that inhibit critical biological mechanisms of restenosis. These stents are termed drug-eluting stents (DES) (4). Currently, two devices are approved by the FDA, both of which have shown efficacy towards the prevention of restenosis, the Cypher™ stent (Cordis, Johnson&Johnson), which is coated with sirolimus (5) and the Taxus^{®} stent (Boston Scientific) (6) that releases paclitaxel. To protract the release of the cytostatic compound, which is critical for the clinical success of a drug-eluting stent, both devices use a polymeric coating to retard drug release. While this choice of coating is effective, it imposes several risks which may limit the clinical outcome (7). Polymers are known to induce inflammatory responses (8,9) which can translate into delayed vascular healing and thus increased risk for an adverse outcome (7). In deceased patients who previously received a polymer coated drug-eluting stent, ongoing inflammation and impeded enothelialization could be observed consistently (10,11). As it is now becoming evident, the rate of late stent thrombosis which has a fatal outcome in up to 40% of patients (12) may be increased in patients that received a drug-eluting stent (evidence from the BASKET-LATE trial, as presented at the annual meeting of the American College of Cardiology, Atlanta, GA, USA, March 2006). Furthermore, polymeric stent coating may lead to late in-stent restenosis (13,14) which hampers the beneficial long-term effects of current polymer-based DES platforms.

To circumvent the need for polymeric coating, we recently introduced a drug-eluting stent platform that uses a microporous stent surface that serves as "drug pockets" to delay the release of a given drug (15). The system proved safe and effective both in a standard pre-clinical model (15) as well as in humans (16,17). However, in certain clinical situations a slower release kinetic might be required. Since both stents are loaded with comparable dosages of the identical drug, it is highly suggestive that the protracted drug release of the Cypher™ stent, which liberates the drug within 60 days, is responsible for the superior clinical results concerning in-stent restenosis at 6-9 months follow up. Therefore, to improve clinical performance of a stent that is intended to be used as a drug-eluting stent, we identified a novel method that is based on the use of natural products to retard the release of the stent-based anti-restenotic compound by simultaneous coating with one or several natural products. The anti-restenotic compound can be a drug, a nucleic acid or other substances such as gene vectors that can influence the pathophysiology of restenosis.

### 2. Methods

### Stent coating

Currently, we apply spray-coating by means of a stent-coating machine (15) to coat vascular stents. However, other coating processes may be applied to coat the stent appropriately with natural products. The natural product is provided as a mixture together with the compound but can also be applied separately on the stent surface to alter the release of the compound. To show proof-of-principle, we selected rapamycin as the compound for protracted release by means of natural products. Currently, rapamycin is regarded as the most effective anti-restenotic compound used on drug-eluting stent platforms (18). Time-dependent rapamycin release from the stent platform was measured at distinctive time points as previously described (15) by means of UV spectroscopy at a wave length of 280 nm. Beforehand, it was confirmed that the UV spectres of the natural products did not interfere with the one from rapamycin.

### Natural products

Natural products are substances that occur naturally in contrast to those that are chemically synthesised. For the present purpose, natural products are selected from waxes andresins, especially shellac. To show proof-of-principle, we intensively investigated the use of shellac to coat drug-eluting stents. Careful assessment of the dosage that is optimal for stent coating revealed a concentration of 0.5% shellac dissolved in ethanol p.a. (pro analysi).

### 3. Results

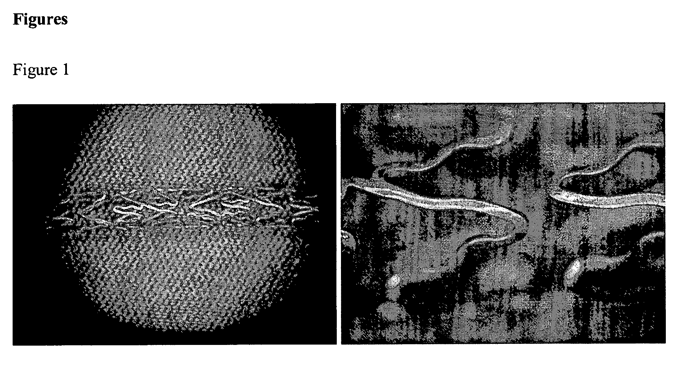

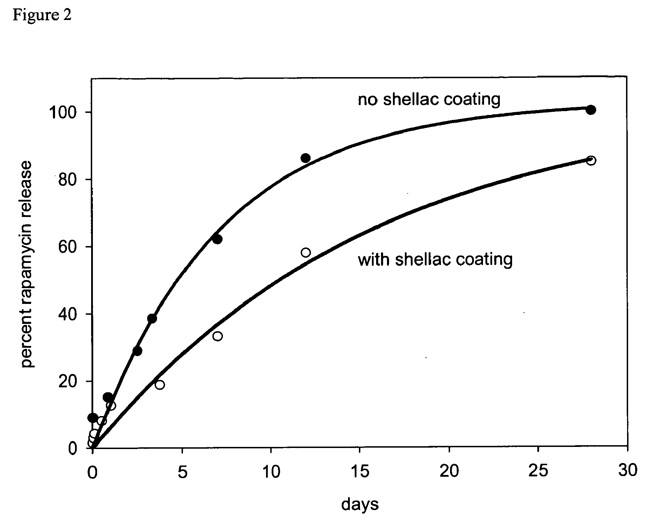

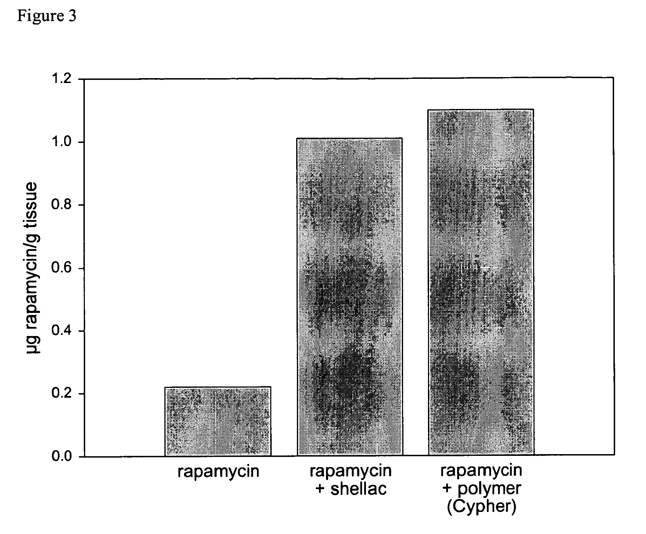

Shellac was either used as a separate coating layer or applied as a mixture with distinct dosages of rapamycin on the stent surface. The appearance of the stent surface is depicted in Fig.1. As apparent, no webbing or clumping which is described for the current FDA-approved DES platforms (19,20) and that can be associated with adverse events occurred with shellac coating. Importantly, drug release was significantly retarded by the use of shellac, as shown in Fig.2. Stent implantation into porcine coronary arteries, which is considered the standard animal model for preclinical evaluation of novel stent platforms, showed no apparent adverse effect of shellac on critical determinants of vascular integrity and retarded drug release with elevated tissue concentrations of rapamycin three weeks after stent placement (Fig.3). The amount of drug that could be identified in the stented vascular wall was similar to the amount measured after placement of the FDA-approved rapamycin-coated, polymer based Cypher™ stent, thus proving that drug release can be effectively protracted by the usage of this natural product.

### 4. Discussion

Potential advantages of drug-eluting stents coated with natural products to protract drug release

The potential advantages of coating with natural products are obvious: retardation of primary drug release without the acute and/or chronic presence of a polymeric release modulator that imposes potentially dangerous vascular reactions and potential adverse outcomes such as life threatening stent thrombosis and/or late in-stent restenosis. Thus, the proposed novel coatings of drug-eluting stents with a natural product or with natural products are likely to improve both efficacy as well as safety of drug-eluting stents.

References
1. Topol EJ, Serruys PW. Frontiers in interventional cardiology. Circulation 1998;98:1802-1820.
2. Serruys PW, de Jaegere P, Kiemeneij F. A comparison of balloon-expandable stent implantation with balloon angioplasty in patients with coronary artery disease. N Engl J Med 1994;331:489-495.
3. Kastrati A, Mehilli J, Dirschinger J, et al. Restenosis after coronary placement of various stent types. Am J Cardiol 2001;87:34-39.
4. Kastrati A, Dibra A, Eberle S, et al. Sirolimus-eluting stents vs paclitaxel-eluting stents in patients with coronary artery disease: meta-analysis of randomized trials. JAMA 2005;294:819-825.
5. Moses JW, Leon MB, Popma JJ, et al. Sirolimus-eluting stents versus standard stents in patients with stenosis in a native coronary artery. N Engl J Med 2003;349:1315-1323.
6. Stone GW, Ellis SG, Cox DA, et al. A polymer-based, paclitaxel-eluting stent in patients with coronary artery disease. N Engl J Med 2004;350:221-231.
7. Joner M, Finn AV, Farb A, et al. Pathology of drug-eluting stents in humans: delayed healing and late thrombotic risk. J Am Coll Cardiol 2006;in press.
8. Goodwin SC, Yoon H-C, Chen G, et al. Intense inflammatory reaction to heparin polymer coated intravascular Palmaz stents in porcine arteries compared to uncoated Palmaz stents. Cardiovasc Interven Radiol 2003;26:158-167.
9. van der Giessen WJ, Lincoff AM, Schwartz RS, et al. Marked inflammatory sequelae to implantation of biodegradable and nonbiodegradable polymers in porcine coronary arteries. Circulation 1996;94:1690-7.
10. Virmani R, Guagliumi G, Farb A, et al. Localized hypersensitivity and late coronary thrombosis secondary to a sirolimus-eluting stent: should we be cautious? Circulation 2004;109:701-705.
11. Farb A, Burke AP, Kolodgie FD, Virmani R. Pathological Mechanisms of Fatal Late Coronary Stent Thrombosis in Humans. Circulation 2003;108:1701-1706.
12. Iakovou I, Schmidt T, Bonizzoni E, et al. Incidence, predictors, and outcome of thrombosis after successful implantation of drug-eluting stents. JAMA 2005;293:2126-2130.
13. Wessely R, Kastrati A, Schömig A. Late restenosis in patients receiving a polymer coated sirolimus-eluting stent. Ann Intern Med 2005;143:392-4.
14. Valgimigli M, Malagutti P, van Mieghem CAG, et al. Persistence of neointimal growth 12 months after intervention and occurrence of delayed restenosis in patients with left main coronary artery disease treated with drug-eluting stents. J Am Coll Cardiol 2006;47:1491-1494.
15. Wessely R, Hausleiter J, Michaelis C, et al. Inhibition of neointima formation by a novel drug-eluting stent system that allows for dose-adjustable, multiple, and on-site stent coating. Arterioscler Thromb Vasc Biol 2005;25:748-753.
16. Hausleiter J, Kastrati A, Wessely R, et al. Prevention of restenosis by a novel drug-eluting stent system with a dose-adjustable, polymer-free, on-site stent coating. Eur Heart J 2005;26:1475-1481.
17. Mehilli J, Kastrati A, Wessely R, et al. Randomized trial of a nonpolymer-based rapamycin-eluting stent versus a polymer-based paclitaxel-eluting stent for the reduction of late lumen loss. Circulation 2006; 113:273-79.
18. Wessely R, Schömig A, Kastrati A. Sirolimus and paclitaxel on polymer-based drug-eluting stents: similar but different. J Am Coll Cardiol 2006;47:708-14.
19. Roffi M, Luscher TF, Sutsch G, et al. Failure to retrieve undeployed paclitaxel-eluting coronary stents. Am J Cardiol 2006;97:502-505.
20. Regar E, Sianos G, Serruys PW. Stent development and local drug delivery. Br Med Bull 2001;59:227-248.

### Example 2

The purpose of this study was to evaluate the effects of a stent coating with poly.DL-lactide alone or in combination with shellac.

The coating procedure was as described in Example 1. First, stents were coated with a solution containing 0.4 % rapamycin (4 mg/ml) and 0.7 % poly-DL-lactide (Resomer R 202 S, Boehringer Ingelheim) (7 mg/ml) in ethyl acetate or with a solution containing rapamycin alone in ethyl acetate. For the experiments with a combination of poly-DL-lactide and shellac, the stents were then further coated with a solution of 0.1 % rapamycin (1 mg/ml) and 0.5 % shellac (5 mg/ml) in ethanol.

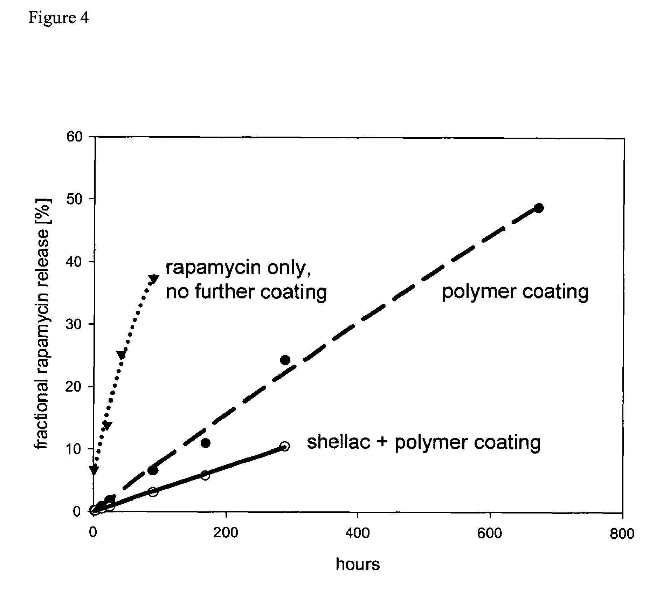

The effects were evaluated as described in Example 1. As it can be taken from Figure 4, the coating with poly-DL-lactide alone already resulted in a significant retardation of drug release, which was even increased when poly-DL-lactide and shellac were co-coated.

## Claims

1. An implant or a part thereof coated with a wax or a resin.

2. The implant or part thereof of claim 1, wherein the wax is selected from the group consisting of plant derived, animal derived and geologically derived waxes.

3. The implant or part thereof of claim 1, wherein the resin is shellac.

4. The implant or part thereof of any of claims 1 to 3, wherein the implant is further coated with polylactide, preferably poly-DL-lactide.

5. The implant or part thereof of any of claims 1 to 4, wherein the implant comprises metal.

6. The implant or part thereof of any of claims 1 to 5, wherein the implant is a valve, a dental implant or an orthopaedic implant system.

7. The implant or part thereof of any of claims 1 to 5, wherein the implant is a stent.

8. The implant or part thereof of any of claims 1 to 7, wherein the implant is further coated with a compound.

9. The implant or part thereof of claim 8, wherein the compound is a drug.

10. The implant or part thereof of claim 9, wherein the drug is rapamycin, everolimus, pimecrolimus, paclitaxel, or zotaolismus, or an analogue thereof, or a statin, an immunosuppressive drug or an antiproliferative drug.

11. A method of preparing the implant according to any of claims 1 to 7, wherein an uncoated implant or part thereof is coated with the wax or the resin.

12. The method of claim 11, wherein the implant or part thereof is further coated with a compound, preferably with a drug, and more preferably with a drug as defined in claim 10.

13. The method of any of claims 11 or 12, wherein the uncoated implant or part thereof is spray-coated with the wax or resin.

14. The method of any of claims 12 or 13, wherein the implant or part thereof is further spray-coated with the drug.

15. The implant of any of claims 1 to 10 for use in the prophylaxis or therapy of a disease, preferably a cardiac or vascular disease, more preferably a stenosis or restenosis.

16. A method of treating a subject, wherein an implant according to any of claims 1 to 10 is implanted into a subject.

17. The method of claim 16 wherein the subject is suffering from a cardiac or vascular disease, more preferably a stenosis or restenosis.
